# EUROPÄISCHE PATENTSCHRIFT

(11) **EP 1 407 796 B1**
(45) Veröffentlichungstag und Bekanntmachung des Hinweises auf die Patenterteilung: **27.01.2010**
(21) Anmeldenummer: 03022359.8
(22) Anmeldetag: 04.10.2003
(51) Int. Cl.: A61M 16/04

(54) **Arretiereinrichtung**
Locking device
Dispositif de verrouillage

(30) Priorität: 08.10.2002 DE 10246931
(43) Veröffentlichungstag der Anmeldung: 14.04.2004
(73) Patentinhaber: Willy Rüsch GmbH, 71394 Kernen-Rommelshausen (DE)
(72) Erfinder: Wurster, Helmut, 75038 Oberdingen (DE); Rak, Karl, 73614 Schorndorf (DE); Singvogel, Armin, 71686 Remseck (DE)
(74) Vertreter: Grünecker, Kinkeldey, Stockmair & Schwanhäusser Anwaltssozietät

(56) Entgegenhaltungen:
- WO-A-95/20419
- WO-A-97/31669
- DE-C- 824 688
- FR-A- 1 087 344
- FR-A- 1 426 926
- US-A- 4 850 348
- US-A- 5 052 386

## Beschreibung

### Stand der Technik

Die Erfindung betrifft eine Arretiereinrichtung mit vom Patienten abgewandten Klemmbacken, die eine Führung für eine Tracheostomiekanüle bilden, wobei die Klemmbacken zum Arretieren der Arretiereinrichtung an der Tracheostomiekanüle mit einem Spannelement zusammen wirken.

Tracheostomiekanülen sind Tuben, über die eine Verbindung der Trachea nach außen hergestellt wird. Am patientenabgewandten Ende der Tracheostomiekanüle befindet sich in der Regel ein Schild, durch dessen Anlage am Hals des Patienten das patientenabgewandte Ende der Tracheostomiekanüle fixiert wird.

Um die Tracheostomiekanüle bei Patienten verschiedener Körpergröße einsetzen zu können, ist es vorteilhaft, wenn das Schild entlang der Tracheostomiekanüle verschoben werden kann, so dass eine individuelle Anpassung an den zu behandelnden Patienten erfolgen kann. Dazu ist es bekannt, am Schild auf der patientenabgewandten Seite ein geschlitztes Rohr auszubilden, in dem die Tracheostomiekanüle geführt ist. Die durch Einschnitte getrennten Abschnitte des Rohres bilden Klemmbacken. Sie liegen flächig an der Tracheostomiekanüle an. Die Klemmbacken werden durch eine Ringmutter gegen die Tracheostomiekanüle gedrückt, wobei insbesondere die freien Enden der Klemmbacken gegen die Tracheostomiekanüle drücken, so dass es zu einer Klemmverbindung in Umfangsrichtung der Tracheostomiekanüle kommt.

In der US 5,251,616 ist beschrieben, ein Halteelement zwischen einem mit dem Schild verbundenen Verschlusselement und einer Ringmutter derart zu verklemmen, dass die Tracheostomiekanüle eingeklemmt und an dem Schild fixiert wird.

Die US 4,235,229 offenbart eine Arretiereinrichtung für eine Tracheostomiekanüle mit einem elastisch verformbaren Spannelement mit zwei Klemmbacken, die innerhalb einer ovalen Öffnung des Spannelements angeordnet sind, derart, dass die Klemmbacken eine zwischen den Klemmbacken angeordnete Tracheostomiekanüle Festklemmen. Das Spannelement ist an einem Flansch zur Fixierung der Tracheostomiekanüle am Patienten, angeordnet. Die ovale Öffnung weist einen schmalen und einen breiten Durchmesserbereich auf, wobei die Klemmbacken im schmalen Durchmesserbereich angeordnet sind. Wird das Spannelement in Richtung des breiten Durchmesserbereichs zusammengedrückt, so wird das Festklemmen der Tracheostomiekanüle gelockert und diese kann durch die Öffnung verschoben werden.

Die WO 95/20419 offenbart eine verschieb sichere Befestigung, die jedoch nicht verstellbar ist.

Während die zuvor beschriebenen Befestigungsarten zur unverschieblichen Fixierung des Schilds auf der Tracheostomiekanüle bei starren Tracheostomiekanülen hinreichend gute Ergebnisse liefern, kann eine verschiebsichere Befestigung an flexiblen Tracheostomiekanülen nicht sicher gestellt werden. Werden die Abschnitte durch die Ringmutter gegen die Tracheostomiekanüle verklemmt, gibt die Außenwand der Tracheostomiekanüle nach, so dass das Schild verschoben werden kann. Insbesondere bei flexiblen Tracheostomiekanülen, die von einer Wendel ummantelt sind, ist eine verschiebesichere Befestigung kaum möglich.

### Aufgabe der Erfindung

Aufgabe der vorliegenden Erfindung ist es deshalb, eine Befestigungsvorrichtung zu schaffen, mit der ein Schild an einer Tracheostomiekanüle in verschiedenen Stellungen unverschieblich gehalten werden kann.

### Gegenstand der Erfindung

Erfindungsgemäß wird diese Aufgabe dadurch gelöst, dass bei der eingangs genannten Arretiereinrichtung mindestens eine Klemmbacke unter Einfluss des Spannelements verwindbar ist und eine axiale Anlagefläche aufweist, wobei die Anlagefläche im verwundenen Zustand in geringerem Abstand zur Mittelachse der Führung angeordnet ist als im verwindungsfreien Zustand. Im nicht verspannten Zustand kann die Arretiereinrichtung ungestört entlang der Tracheostomiekanüle verschoben werden. Im arretierten Zustand bewirkt das Spannelement eine Verwindung der Klemmbacke. Eine Anlagefläche der Klemmbacke klemmt die Tracheostomiekanüle fest. Durch die Anlagefläche werden mehrere Windungen einer die Tracheostomiekanüle ummantelnden Wendel erfasst. Im Gegensatz zum Stand der Technik, bei dem die Klemmbacken nur in Umfangsrichtung zwischen zwei Wendelwindungen eingreifen, erstreckt sich erfindungsgemäß die die Klemmung verursachende Anlagefläche quer zu den Wendelwindungen und erfasst je nach Wendelsteigung mehrere Windungen. Insbesondere eine flexible, spiralarmierte Tracheostomiekanüle wird damit verrutschsicher eingeklemmt. Durch eine geringere Steigung der Wendelwindungen kann die Verrutschsicherung noch erhöht werden, da damit die Anzahl der Windungen einer die Tracheostomiekanüle ummantelnden Wendel erhöht wird, welche von den Klemmbacken erfasst werden.

Bei einer bevorzugten Ausführungsform verjüngt sich mindestens eine Klemmbacke zumindest in einem axialen Abschnitt in Umfangsrichtung der Tracheostomiekanüle. Wird von dem Spannelement von außen auf die Klemmbacke gedrückt, verwindet sich die Klemmbacke umso mehr, je näher der Druck am dickeren Ende der Klemmbacke ausgeübt wird. Unter dem Druck des Spannelements verwindet sich die Klemmbacke, so dass die dickere Seite der Klemmbacke, an der sich vorzugsweise die axiale Anlagefläche befindet, auf der Außenoberfläche der Tracheostomiekanüle rutschfest aufliegt. Durch diese Maßnahme verhakt sich die Klemmbacke in der Tracheostomiekanüle. Die Arretiereinrichtung wird unverschieblich auf der Tracheostomiekanüle gehalten. Die erfindungsgemäße Arretiereinrichtung kann daher besonders vorteilhaft bei flexiblen Tracheostomiekanülen eingesetzt werden. Somit kann die Position der Arretiereinrichtung an der Tracheostomiekanüle stufenlos verändert werden. Die Tracheostomiekanüle kann bei Patienten verschiedener Körpergröße eingesetzt werden.

Wenn der sich verjüngende Abschnitt am freien Ende der Klemmbacke angeordnet ist, kann eine starke Verwindung der Klemmbacke erreicht werden. Sind mehrere, insbesondere drei Klemmbacken vorgesehen, kann die Trachesotomiekanüle besonders gut eingeklemmt werden.

Bei einer besonders bevorzugten Ausführungsform weisen die Klemmbacken eine Krümmung auf, die im Wesentlichen der Krümmung der Oberfläche der Tracheostomiekanüle entspricht. Im nicht verspannten Zustand kann die Arretiereinrichtung besonders gut entlang der Tracheostomiekanüle gleiten. Bereits im nicht verspannten Zustand liegen die Klemmbacken ohne einen wesentlichen Anpressdruck an der Tracheostomiekanüle an. Werden die Klemmbacken mit dem Spannelement verspannt, so geraten die dicken Enden der Abschnitte sofort in Eingriff mit der Tracheostomiekanüle.

Besonders vorteilhaft ist es, wenn zumindest die verwindbare Klemmbacke eine oder mehrere Materialausnehmungen aufweist. Wenn die Klemmbacke an einer oder mehreren Stellen weniger Material oder gar kein Material, also dünnere Materialdicken, Dellen oder Löcher aufweist, dann kann eine Klemmbacke besonders flexibel ausgestaltet werden. Durch das Spannelement muss daher nur eine geringe Kraft auf die Klemmbacke ausgeübt werden, um eine verrutschsichere Befestigung der Arretiereinrichtung an der Tracheostomiekanüle zu bewirken.

Vorzugsweise sind die Klemmbacken aus Hartkunststoff gefertigt. Beim Verspannen greift das harte Material der verbundenen Klemmbacken an relativ weichen Materialabschnitten der Tracheostomiekanüle an, wodurch eine besonders gute Befestigung der Arretiereinrichtung an der Tracheostomiekanüle erfolgt.

Bei einer bevorzugten Ausführungsform ist das Spannelement an der Arretiereinrichtung unverlierbar gehalten. Durch diese Maßnahme kann die Arretiereinrichtung entlang der Tracheostomiekanüle in die richtige Position verschoben werden. Zum Befestigen der Arretiereinrichtung in der ausgewählten Position ist nur das Verspannen des Spannelements notwendig. Das Spannelement muss nicht extra aufgesetzt werden. Außerdem ist es nicht möglich, das Spannelement zu verlieren. Vorzugsweise ist im Spannelement eine Ringnut vorgesehen, in die eine Nase der Arretiereinrichtung eingreift. Dadurch kann das Spannelement unverlierbar aber verdrehbar an der Arretiereinrichtung gehalten werden.

Wenn das Spannelement gegenüber der Arretiereinrichtung verdrehbar ist, insbesondere um 90° verdrehbar ist, kann der Eingriff der Klemmbacken in die Tracheostomiekanüle besonders einfach bewirkt werden. Durch eine Verdrehung des Spannelements in Richtung der Verdickung der Klemmbacken wird eine rutschfeste Anlage der Klemmbacken an der Außenwandung der Tracheostomiekanüle besonders einfach und schnell bewirkt. Gelöst wird die Arretiereinrichtung ebenso einfach und schnell durch Drehung in entgegengesetzter Drehrichtung.

Ist ein Anschlag für das Spannelement vorgesehen, so kann ein Überdrehen des Spannelements verhindert werden. Durch diese Maßnahme wird eine übermäßige Verengung der Tracheostomiekanüle verhindert und das unbeschwerte Atmen des Patienten sichergestellt.

Wenn das Spannelement mindestens einen radial nach innen vorstehenden Vorsprung, insbesondere der Anzahl der Klemmbacken entsprechende radial nach innen vorstehende Vorsprünge aufweist, dann bewegen sich beim Verspannen des Spannelements die Vorsprünge entlang der Außenoberfläche der sich verjüngenden Abschnitte der Klemmbacken. Je weiter das Spannelement verdreht wird, desto stärker drücken die Backen auf die Außenseite der Klemmbacken und bewirken deren Verwindung. Die Klemmbacken weichen den Vorsprüngen aus, so dass es zu einer festen Anlage der Klemmbacken an der Tracheostomiekanüle kommt. Die Arretiereinrichtung wird dadurch unverschieblich gehalten. Durch die Verwindung der Klemmbacken bewegen sich die dünnen Seiten der Klemmbacken radial nach außen. Auf.diese Weise bilden sie einen Anschlag für die Vorsprünge, die radial auf benachbarte Klemmbacken drücken, so dass ein Überdrehen des Spannelements verhindert wird. In der verspannten Stellung erfolgt ein Einrasten, so dass eine sichere Arretierung gewährleistet ist. Bei geöffneter Arretiereinrichtung schlagen die Vorsprünge an den dicken Seiten der sich verjüngenden Abschnitte der Klemmbacken an. Somit gibt es eine definierte Offen- und Klemmstellung des Spannelements.

Vorteilhafterweise weist das Spannelement eine Durchführungsöffnung für die Tracheostomiekanüle auf und hat ebenso viele Spannbacken wie das Gegenstück Klemmbacken besitzt. Die Form der vorspringenden Vorsprünge ist an die Verjüngung bzw. an die Verdickung der Klemmbacken angepasst. Beim Verdrehen des Spannelements in Richtung Verdickung der Klemmbacken wird bewirkt, dass die Klemmbacken radial nach innen ausgelenkt und in sich verdreht werden.

Bei einer bevorzugten Ausführungsform ist ein Schild an der Arretiereinrichtung befestigbar. Durch diese Maßnahme kann die erfindungsgemäße Arretiereinrichtung zum Befestigen von einem Schild an der Tracheostomiekanüle verwendet werden. Das Schild kann je nach Körpergröße des Patienten in die richtige Position an der Tracheostomiekanüle gebracht werden und mittels der Arretiereinrichtung in dieser Position arretiert werden.

Bei einer vorteilhaften Weiterbildung ist die Arretiereinrichtung einstückig mit dem Schild ausgebildet. Aufgrund der einstückigen Ausbildung ist die Anordnung besonders stabil. Außerdem kann sie besonders kostengünstig hergestellt werden. Eine einstückige Ausbildung erleichtert zudem die Reinigung.

Weitere Merkmale und Vorteile der Erfindung ergeben sich aus der nachfolgenden Beschreibung eines Ausführungsbeispiels der Erfindung, anhand den Figuren der Zeichnung, die erfindungswesentliche Einzelheiten zeigen, und aus den Ansprüchen. Die einzelnen Merkmale können je einzeln für sich oder zu mehreren in beliebiger Kombination bei einer Variante der Erfindung verwirklicht sein.

### Zeichnung

Ein Ausführungsbeispiel der erfindungsgemäßen Arretiereinrichtung ist in der schematischen Zeichnung dargestellt und wird in der nachfolgenden Beschreibung erläutert. Es zeigt:
- Fig. 1: eine Draufsicht auf das freie Ende der erfindungsgemäßen Arretiereinrichtung;
- Fig. 2: eine Draufsicht auf ein Spannelement;
- Fig. 3: einen Längsschnitt der Arretiereinrichtung gemäß III-III in Fig. 1;
- Fig. 4a: eine Draufsicht auf eine Arretiereinrichtung mit geöffnetem Spannelement; und
- Fig. 4b: eine Draufsicht auf eine Arretiereinrichtung mit verdrehtem Spannring.

### Beschreibung des Ausführungsbeispiels

Die Figuren sind sehr schematisch dargestellt, um die wesentlichen erfinderischen Merkmale zu verdeutlichen. In den Darstellungen sind die Dimensionen nur beispielhaft und nicht maßstäblich zu verstehen.

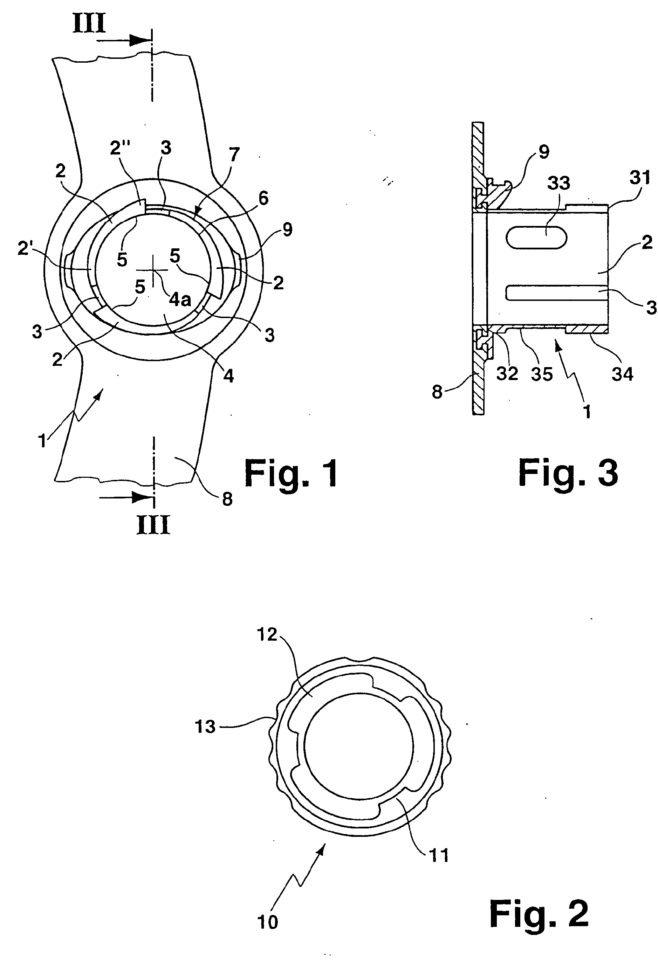

**Fig. 1** zeigt eine Draufsicht auf das freie Ende der Arretiereinrichtung **1**. Die Arretiereinrichtung 1 weist Klemmbacken **2** auf, die durch einen Schlitz **3** voneinander beabstandet sind. Die Klemmbacken 2 bilden zusammen eine Führung **4**, in der eine Tracheostomiekanüle geführt werden kann. Die Klemmbacken 2 weisen eine axiale Anlagefläche **5** auf, mit der in verspanntem Zustand die Trachesotomiekanüle eingeklemmt wird. Die der Führung 4 zugewandte Seite **6** der Klemmbacken 2 ist an die Krümmung der Tracheostomiekanüle angepasst. Die gekrümmten Seiten 6 liegen auf einer Kreisbahn um die Mittelachse **4a** der Führung 4. Die Klemmbacken 2 verjüngen sich in Umfangsrichtung **7**, so dass sie ein dünnes Ende 2' und ein dickes Ende 2" aufweisen, wobei die Kreisbahn um die Mittelachse 4a unverändert bleibt. Die Verjüngung wird durch eine Veränderung des Außendurchmessers bezogen auf die Mittelachse 4a erreicht (Materialabnahme nur an der Außenoberfläche). Die Arretiereinrichtung 1 ist an einem Schild **8**, welches zur Anlage an den Hals eines Patienten bestimmt ist, befestigt. Es sind zwei gegenüber liegende Nasen **9** vorgesehen, die in eine Ringnut eines in Fig. 2 dargestellten Spannelements greifen. Das Spannelement wird auf die Arretiereinrichtung aufgesetzt, indem das Spannelement so radial zusammen gedrückt und deformiert wird, dass es über die Nasen 9 passt. Wird das Spannelement los gelassen, greifen die Nasen 9 in die Ringnut und halten das Spannelement unverlierbar.

**Fig. 2** zeigt eine Draufsicht auf ein Spannelement **10**, welches dazu benutzt werden kann, die Klemmbacken der Arretiereinrichtung zu verspannen. Das Spannelement 10 weist radial nach innen stehende Vorsprünge **11** auf. In die Zwischenräume **12** zwischen den Vorsprüngen 11 können sich die dünnen Enden der Klemmbacken bewegen, wenn diese sich unter dem Druck der Vorsprünge 11 auf die dicken Enden der Klemmbacken verwinden. Die dünnen Enden der Klemmbacken bilden dann einen Anschlag für einen Vorsprung 11. Im unverspannten Zustand sind die dünnen Enden im Wesentlichen unter den Vorsprüngen 11 und die dicken Enden der Klemmbacken in den Zwischenräumen 12 angeordnet. Am Außenumfang des Spannelements 10 sind Mulden **13** vorgesehen. Die Mulden 13 verhindern beim Drehvorgang des Spannelements 10 ein Abrutschen des Benutzers. Bei Verdrehen des auf die Klemmbacken aufgesetzten Spannelements 10 entgegen der Pfeilrichtung 7 (siehe Fig. 1) gleiten die Vorsprünge 11 entlang der Außenoberfläche der Klemmbacken. Je weiter die Vorsprünge 11 entlang der Klemmbacken gleiten, desto weiter wird das dicke Ende der Klemmbacken radial nach innen bewegt. Dabei führt die dicke Seite am freien Ende der Klemmbacken eine stärkere Bewegung radial nach innen aus, als eine Stelle am dem freien Ende gegenüberliegenden Ende der Klemmbacke. Entsprechend bewegt sich das dünne Ende der Klemmbacken am freien Ende stärker radial nach außen als am gegenüberliegenden Ende der Klemmbacke. Dies bewirkt eine Verwindung der Klemmbacke und einen Eingriff der axialen Anlageflächen der Klemmbacken in die Außenoberfläche einer nicht dargestellten Tracheostomiekanüle.

**Fig. 3** zeigt einen Längsschnitt der Arretiereinrichtung 1 gemäß der Linie III-III der Fig. 1, wobei zusätzlich eine von zwei Nasen 9 um 90° gedreht gezeichnet ist. Die Arretiereinrichtung 1 ist an ihrem dem freien Ende **31** gegenüberliegenden Ende **32** mit dem Schild 8 materialschlüssig verbunden. Die Klemmbacken 2 sind durch Schlitze 3 voneinander beabstandet und weisen Materialausnehmungen **33** auf, die die Verwindungsfähigkeit der Klemmbacken 2 erhöhen. Die Materialausnehmung 33 ist als Langloch ausgebildet. Es liegt im handwerklichen Können des Fachmanns, die Materialausnehmung so zu gestalten, dass die gewünschte Flexibilität der Klemmbacke 2 entsteht. Die Klemmbacken 2 weisen an ihrem freien Ende 31 einen axialen Abschnitt **34** auf, der sich in Umfangsrichtung verjüngt. An den axialen Abschnitt 34 schließt sich ein Abschnitt **35** mit gleichmäßiger dünnerer Materialdicke an. Diese Abschnitte 35 aller Klemmbacken 2 bilden im Wesentlichen einen Hohlzylinder. Weiterhin ist eine Nase 9 vorgesehen, die in eine umlaufende Ringnut des Spannelements eingreift und dieses unverlierbar auf der Arretiereinrichtung 1 hält.

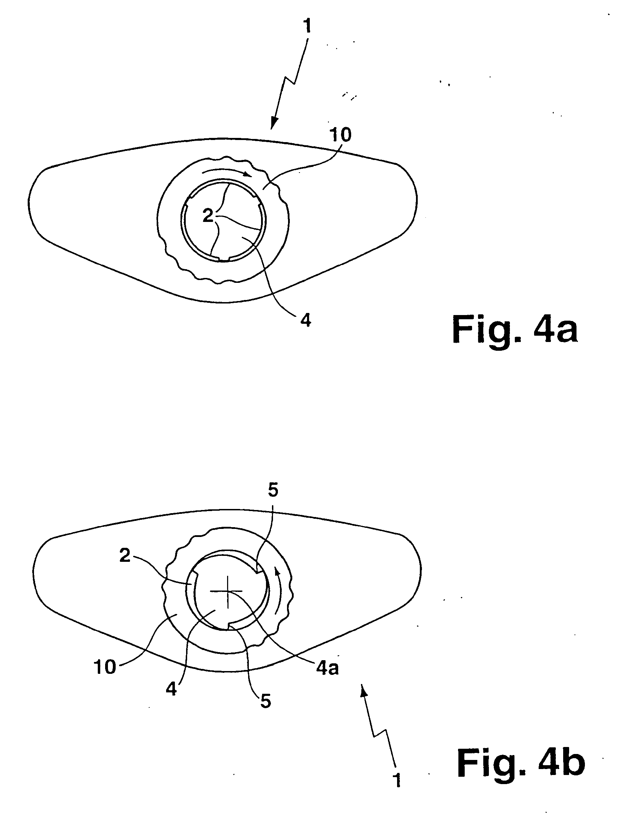

**Fig. 4a** zeigt eine Draufsicht auf die Arretiereinrichtung 1 mit geöffnetem Spannelement 10. Die Klemmbacken 2 sind in nicht verwundenem Zustand und geben die Führung 4 vollständig frei. Das Spannelement 10 kann in Pfeilrichtung verdreht werden.

**Fig. 4b** zeigt die Arretiereinrichtung 1 mit um 90° verdrehtem Spannelement 10. Die Klemmbacken 2 sind verwunden. Die Anlageflächen 5 sind in der Führung 4 in Richtung der Mittelachse 4a der Führung 4 bewegt. Sie haben zur Mittelachse 4a einen geringeren Abstand als im verwindungsfreien Zustand.
Die Klemmbacken 2 decken zwar etwa einen Winkelbereich von 120° ab; da die Vorsprünge des Spannrings 10 jedoch in geöffnetem Zustand einenends an eine erste Klemmbacke anstoßen und im verspannten Zustand anderenends an eine zweite benachbarte Klemmbacke anstoßen und einen Winkelbereich von etwa 30° abdecken, ist der Spannring 10 im Zusammenspiel mit den Klemmbacken 2 so ausgelegt, dass eine Drehbewegung von nur etwa 90° ausreichend ist, die maximale Verspannung an der Arretiereinrichtung 1 zu erzeugen. In Pfeilrichtung kann die Verspannung aufgehoben werden.

Bei einer Arretiereinrichtung 1 mit vom Patienten abgewandten Klemmbacken 2, die eine Führung 4 für eine Tracheostomiekanüle bilden, wobei die Klemmbacken 2 zum Arretieren der Arretiereinrichtung 1 an der Tracheostomiekanüle mit einem Spannelement 10 zusammenwirken, ist mindestens eine Klemmbacke 2 unter Einfluss des Spannelements 10 verwindbar. Die Klemmbacke 2 weist eine axiale Anlagefläche 5 auf, wobei die Anlagefläche 5 im verwundenen Zustand in geringerem Abstand zur Mittelachse 4a der Führung 4 angeordnet ist als im verwindungsfreien Zustand. Die Arretiervorrichtung 1 kann dadurch besonders gut unverrückbar an einer flexiblen Tracheostomiekanüle gehalten werden.

## Patentansprüche

1. Arretiereinrichtung (1) zum unverschiebbaren Halten eines Schildes (8) in verschiedenen Stellungen an einer Tracheostomiekanüle mit vom Patienten abgewandten Klemmbacken (2), die eine Führung (4) für die Tracheostomiekanüle bilden, wobei die Klemmbacken (2) zum Arretieren der Arretiereinrichtung (1) an der Tracheostomiekanüle mit einem Spannelement (10) zusammenwirken, **dadurch gekennzeichnet, dass** mindestens eine Klemmbacke (2) unter Einfluss des Spannelements (10) verwindbar ist und eine axiale Anlagefläche (5) aufweist, wobei die Anlagefläche (5) im verwundenen Zustand zum Festklemmen der Tracheostomiekanüle in geringerem Abstand zur Mittelachse (4a) der Führung (4) angeordnet ist als im verwindungsfreien Zustand.

2. Arretiereinrichtung nach Anspruch 1, **dadurch gekennzeichnet, dass** sich die mindestens eine Klemmbacke (2) zumindest in einem axialen Abschnitt (34) in Umfangsrichtung (7) der Tracheostomiekanüle verjüngt.

3. Arretiereinrichtung nach Anspruch 2, **dadurch gekennzeichnet, dass** der sich verjüngende Abschnitt (34) am freien Ende (31) der mindestens einen Klemmbacke (2) angeordnet ist.

4. Arretiereinrichtung nach einem der vorhergehenden Ansprüche, **dadurch gekennzeichnet, dass** die Klemmbacken (2) eine Krümmung aufweisen, die im Wesentlichen der Krümmung der Oberfläche der Tracheostomiekanüle entspricht.

5. Arretiereinrichtung nach einem der vorhergehenden Ansprüche, **dadurch gekennzeichnet, dass** zumindest die verwindbare Klemmbacke (2) eine oder mehrere Materialausnehmungen (33) aufweist.

6. Arretiereinrichtung nach einem der vorhergehenden Ansprüche, **dadurch gekennzeichnet, dass** die Klemmbacken (2) aus Hartkunststoff gefertigt sind.

7. Arretiereinrichtung nach einem der vorhergehenden Ansprüche, **dadurch gekennzeichnet, dass** das Spannelement (10) an der Arretiereinrichtung (1) unverlierbar gehalten ist.

8. Arretiereinrichtung nach einem der vorhergehenden Ansprüche, **dadurch gekennzeichnet, dass** das Spannelement (10) gegenüber der Arretiereinrichtung (1) verdrehbar ist, insbesondere um 90°.

9. Arretiereinrichtung nach einem der vorhergehenden Ansprüche, **dadurch gekennzeichnet, dass** ein Anschlag für das Spannelement (10) vorgesehen ist.

10. Arretiereinrichtung nach einem der vorhergehenden Ansprüche, **dadurch gekennzeichnet, dass** das Spannelement (10) mindestens eine, insbesondere der Anzahl der Klemmbacken (2) entsprechende radial nach innen vorstehende Vorsprünge (11) aufweist.

11. Arretiereinrichtung nach Anspruch 10, **dadurch gekennzeichnet, dass** die Form der Vorsprünge an die Klemmbacken angepasst ist.

12. Arretiereinrichtung nach einem der vorhergehenden Ansprüche, **dadurch gekennzeichnet, dass** ein Schild (8) an der Arretiereinrichtung (1) befestigt ist.

13. Arretiereinrichtung nach Anspruch 12, **dadurch gekennzeichnet, dass** die Arretiereinrichtung (1) einstückig mit dem Schild (8) ausgebildet ist.

14. Arretiereinrichtung nach einem der Ansprüche 10 bis 13, **dadurch gekennzeichnet, dass** der Anschlag für das Spannelement durch das dünne Ende der Klemmbacken gebildet ist, an den die Vorderkante der Vorsprünge in der verspannten Stellung anschlägt.

## Claims

1. Locking device (1) for non-displaceable holding of a plate (8) in different positions on a tracheostomy cannula, comprising clamping jaws (2) facing away from the patient and forming a guide (4) for the tracheostomy cannula, wherein the clamping jaws (2) cooperate with a tensioning element (10) in order to lock the locking device (1) to the tracheostomy cannula, **characterised in that** at least one clamping jaw (2) is twistable under the influence of the tensioning element (10) and comprises an axial contact surface (5), wherein, for clamping the tracheostomy cannula, in the twisted condition, the contact surface (5) is arranged at a smaller distance from the central axis (4a) of the guide (4) than in the untwisted condition.

2. Locking device according to claim 1, **characterised in that** the at least one clamping jaw (2) narrows at least in an axial section (34) in the peripheral direction (7) of the tracheostomy cannula.

3. Locking device according to claim 2, **characterised in that** the narrowing section (34) is arranged at the free end (31) of the at least one clamping jaw (2).

4. Locking device according to one of the preceding claims, **characterised in that** the clamping jaws (2) have a curvature which substantially corresponds to the curvature of the surface of the tracheostomy cannula.

5. Locking device according to one of the preceding claims, **characterised in that** at least the twistable clamping jaw (2) has one or more material cut-outs (33).

6. Locking device according to one of the preceding claims, **characterised in that** the clamping jaws (2) are made from hard plastics.

7. Locking device according to one of the preceding claims, **characterised in that** the tensioning element (10) is captively held on the locking device (1)

8. Locking device according to one of the preceding claims, **characterised in that** the tensioning element (10) is rotatable relative to the locking device (1), in particular through 90°.

9. Locking device according to one of the preceding claims, **characterised in that** a stop is provided for the tensioning element (10)

10. Locking device according to one of the preceding claims, **characterised in that** the tensioning element (10) comprises at least one radially inwardly extending projection (11), and in particular a number thereof corresponding to the number of clamping jaws (2).

11. Locking device according to claim 10, **characterised in that** the form of the projections (11) is adapted to the clamping jaws.

12. Locking device according to one of the preceding claims, **characterised in that** a plate (8) is fastened to the locking device (1).

13. Locking device according to claim 12, **characterised in that** the locking device (1) is configured integrally with the plate (8).

14. Locking device according to one of the claims 10 to 13, **characterised in that** the stop for the tensioning element is formed by the thin end of the clamping jaws, against which the front edge of the projections makes contact in the tensioned position.

## Revendications

1. Dispositif d'arrêt (1) pour immobiliser une plaque (8) dans différentes positions sur une canule de trachéotomie, avec des mâchoires (2) opposées au patient qui forment une guidage (4) pour la canule de trachéotomie, les mâchoires (2) coopérant avec un élément de serrage (10) pour arrêter le dispositif d'arrêt (1) sur la canule de trachéotomie, **caractérisé en ce qu'**au moins une mâchoire (2) est apte être tordue sous l'influence de l'élément de serrage (10) et présente une surface d'application axiale (5), la surface d'application (5) étant disposée à une plus faible distance de l'axe médian (4a) du guidage (4) dans la position tordue destinée au blocage de la canule de trachéotomie, par rapport à la position non tordue.

2. Dispositif d'arrêt selon la revendication 1, **caractérisé en ce que** la ou les mâchoires (2) ont une forme effilée au moins dans une partie axiale (34), dans le sens circonférentiel (7) de la canule de trachéotomie.

3. Dispositif d'arrêt selon la revendication 2, **caractérisé en ce que** la partie effilée (34) est disposée à l'extrémité libre (31) de la mâchoire ou des mâchoires (2).

4. Dispositif d'arrêt selon l'une des revendications précédentes, **caractérisé en ce que** les mâchoires (2) présentent une courbure qui correspond globalement à la courbure de la surface de la canule de trachéotomie.

5. Dispositif d'arrêt selon l'une des revendications précédentes, **caractérisé en ce que** la mâchoire apte à être tordue (2), au moins, présente un ou plusieurs enlèvements de matière (33).

6. Dispositif d'arrêt selon l'une des revendications précédentes, **caractérisé en ce que** les mâchoires (2) sont fabriquées en matière plastique dure.

7. Dispositif d'arrêt selon l'une des revendications précédentes, **caractérisé en ce que** l'élément de serrage (10) est fixé de manière imperdable au dispositif d'arrêt (1) .

8. Dispositif d'arrêt selon l'une des revendications précédentes, **caractérisé en ce que** l'élément de serrage (10) est apte à être tourné par rapport au dispositif d'arrêt (1), en particulier de 90°.

9. Dispositif d'arrêt selon l'une des revendications précédentes, **caractérisé en ce qu'**une butée est prévue pour l'élément de serrage (10).

10. Dispositif d'arrêt selon l'une des revendications précédentes, **caractérisé en ce que** l'élément de serrage (10) présente au moins une saillie (11), en particulier un nombre de saillies (11) dirigées radialement vers l'intérieur qui correspond au nombre de mâchoires (2).

11. Dispositif d'arrêt selon la revendication 10, **caractérisé en ce que** la forme des saillies est adaptée aux mâchoires.

12. Dispositif d'arrêt selon l'une des revendications précédentes, **caractérisé en ce qu'**une plaque (8) est fixée au dispositif d'arrêt (1).

13. Dispositif d'arrêt selon la revendication 12, **caractérisé en ce que** le dispositif d'arrêt (1) est formé d'une seule pièce avec la plaque (8).

14. Dispositif d'arrêt selon l'une des revendications 10 à 13, **caractérisé en ce que** la butée pour l'élément de serrage est formée par l'extrémité mince des mâchoires contre lesquelles bute l'arête avant des saillies dans la position serrée.
